# EUROPÄISCHE PATENTSCHRIFT

(11) **EP 1 598 037 B1**
(45) Veröffentlichungstag und Bekanntmachung des Hinweises auf die Patenterteilung: **02.05.2007**
(21) Anmeldenummer: 05010003.1
(22) Anmeldetag: 09.05.2005
(51) Int. Cl.: A61F 13/496, A61F 13/64, A61F 13/62

(54) **Verschluss für absorbierende Artikel in Rollenform**
Fastener for absorbing articles in form of a role
Fermeture d'article absorbant ayant la forme d'un rôle

(30) Priorität: 18.05.2004 DE 102004024590
(43) Veröffentlichungstag der Anmeldung: 23.11.2005
(73) Patentinhaber: Paul Hartmann AG, 89522 Heidenheim (DE)
(72) Erfinder: Halbauer, Peter, 89547 Gerstetten (DE)
(74) Vertreter: Dreiss, Fuhlendorf, Steimle & Becker

(56) Entgegenhaltungen:
- EP-A- 0 920 847
- EP-A- 0 983 760
- WO-A-98/29081
- US-A1- 2004 082 933

## Beschreibung

Die Erfindung betrifft ein Flachmaterial, insbesondere zur Bildung von Verschlussmitteln bei einem wegwerfbaren Hygieneartikel, mit den Merkmalen des Oberbegriffs des Anspruchs 1. Ein derartiges Flachmaterial ist bekannt aus EP-A-0 920 847.

Verschlussmittel für Hygieneartikel werden oftmals als streifen- oder tapeförmige Abschnitte einer endlosen Flachmaterialbahn gewonnen und in einer schnelllaufenden Maschine auf eine Oberfläche einer Komponente eines Hygieneartikels appliziert. Die Verschlussmittel werden demnach weitgehend außerhalb des Herstellungsprozesses für den Hygieneartikel als Endlosmaterial vorgefertigt und üblicherweise auch als Endlosbahn einer Herstellungsmaschine zugeführt, wo dann Längsabschnitte dieser Endlosbahn abgetrennt und zur Bildung der Verschlussmittel appliziert werden. Solche streifen- oder tapeförmigen Verschlussmittel werden in bekannter Weise zwischen Chassis bildenden Materialien eines Hygieneartikels, also beispielsweise zwischen Topsheet und Backsheet, laminiert oder sie sind selbst Y-förmig ausgebildet und schließen ein Flachmaterial zwischen den Schenkeln der Y-Form ein. Es ist aber auch denkbar, dass solche Verschlussmittel auf eine Oberfläche eines Hygieneartikels in an sich beliebiger Weise aufgebracht und dort zumindest mit einem Bereich unlösbar festgelegt werden.

Es sind auch Flachmaterialien und aus diesen hergestellte Verschlussmittel bei Hygieneartikeln bekannt, die mechanisch wirkende Verschlusselemente, insbesondere in Form von Haken/Schlaufenmaterialien aufweisen, die mit einem in geeigneter Weise ausgebildeten Auftreffbereich an anderer Stelle des Hygieneartikels lösbar schließend zusammenwirken, z.B. WO-A-98/29081, US 2004/0082933 A1.

Der vorliegenden Erfindung liegt die Aufgabe zugrunde, ein neuartiges Flachmaterial bereitzustellen, das sich in wirtschaftlicher Weise für die Verwendung bei der Herstellung von wegwerfbaren Hygieneartikeln eignet und welches in einfacher Weise an einen Hygieneartikel applizierbar ist.

Diese Aufgabe wird ausgehend von einem erfindungsgemäß durch ein Flachmaterial mit den Merkmalen des Anspruchs 1 gelöst.

Erfindungsgemäß ist der die mechanischen Verschlusselemente aufweisende Flachmaterialabschnitt zunächst so an der ersten Trägerschicht orientiert, dass seine mechanisch wirkenden Verschlusselemente bei Betrachtung der Trägerschicht nicht sichtbar sind, weil sie von dem Flachmaterialabschnitt aufgrund der beanspruchten Orientierung verdeckt werden.

Das erfindungsgemäße Flachmaterial vermag mit seinem ersten Bereich, der in Weiterbildung der Erfindung auch eine Kleberbeschichtung aufweisen kann, auf eine erste Oberfläche aufgebracht zu werden und mit der ersten Kleberbeschichtung auf der (späteren) Rückseite oder Unterseite des Flachmaterialabschnitts auf eine zweite Oberfläche appliziert zu werden. In diesem applizierten Zustand verdeckt dann die Trägerschicht die mechanisch wirkenden Verschlusselemente des Flachmaterialabschnitts. Erst nach Entfernen der Trägerschicht von dem Flachmaterialabschnitt werden die mechanischen Verschlusselemente sichtbar, zugänglich und wirksam. Mit der vorliegenden Erfindung wurde ein in besonders vorteilhafter Weise einsetzbares und verwendbares Flachmaterial geschaffen, welches sich zur Bildung von Verschlussmitteln bei wegwerfbaren Hygieneartikeln eignet. Insbesondere bildet das erste Trägermaterial selbst eine Art Applikationshilfe für den die mechanisch wirkenden Verschlusselemente aufweisenden Flachmaterialabschnitt, und andererseits wirkt es zugleich als Abdeckmittel der mechanisch wirkenden Verschlusselemente vor der Ingebrauchnahme.

Es erweist sich aber auch als vorteilhaft, dass der Flachmaterialabschnitt über die Kleberbeschichtung an dem zweiten Bereich lösbar haftet. Diese Kleberbeschichtung kann in vorteilhafter Weise zuvor auf den zweiten Bereich der Trägerschicht aufgebracht werden. Es erweist sich dann als vorteilhaft, dass beim Lösen der Trägerschicht von dem Flachmaterialabschnitt die Kleberbeschichtung überwiegend an diesem zweiten Bereich der Trägerschicht verbleibt.

Dadurch dass die erste Kleberbeschichtung eine größere Haftkraft zwischen dem Flachmaterialabschnitt und einer Oberfläche, insbesondere einer Komponente eines Hygieneartikels, ausbildet als die Haftverbindung zwischen dem Flachmaterialabschnitt und dem zweiten Bereich ist bei der Ablösung der Trägerschicht von dem Flachmaterialabschnitt sicher gewährleistet, dass der Flachmaterialabschnitt an der zuvor applizierten Oberfläche verbleibt.

In Weiterbildung der Erfindung erweist es sich als vorteilhaft, wenn das Flachmaterial oder zumindest die erste Trägerschicht des Flachmaterials biegsam und flexibel beschaffen ist. Solchenfalls ist nämlich die Möglichkeit eröffnet, das Flachmaterial um Kanten herum zu biegen oder zu falten.

Die erste Trägerschicht kann insbesondere von einem Vliesstoff, einer Folie oder einem Vlies/Folien-Verbundwerkstoff gebildet sein.

In Weiterbildung der Erfindung von besonderer Bedeutung wird Schutz beansprucht für ein erfindungsgemäßes Flachmaterial in einer übereinander angeordneten Konfiguration, also in einer Anordnung, wo mehrere Bahnen des Flachmaterials übereinander zu liegen kommen, wobei die erste Kleberbeschichtung des Flachmaterialabschnitts einer Lage auf einer von dem Flachmaterialabschnitt abgewandten Seite der Trägerschicht einer anderen Lage zu liegen kommt. Eine solche Anordnung könnte beispielsweise eine Stapelform bilden, in der mehrere Längsabschnitte des Flachmaterials übereinander angeordnet sind. Im Hinblick auf eine maschinelle Fertigung erweist es sich aber als vorteilhaft, wenn das Flachmaterial in Endlosform und auf eine Rolle aufgewickelt ist. Bei einer solchen Anordnung des Flachmaterials erweist es sich als vorteilhaft, wenn die Trägerschicht auf ihrer dem Flachmaterialabschnitt abgewandten Seite eine Release-Beschichtung aufweist, also insbesondere auf dieser Seite oder Oberfläche silikonisiert oder in sonstiger Weise derart behandelt ist, dass die Kleberbeschichtung der darauf zu liegen kommenden Lage zerstörungsfrei und leicht ablösbar ist.

Die Erfindung betrifft des Weiteren eine Wegwerfwindel, insbesondere zur Inkontinentenversorgung, mit einem eine in Umfangsrichtung geschlossene Hüftöffnung der Windel bildenden Hüftgürtel mit den Merkmalen des Anspruchs 8.

Diese erfindungsgemäße Wegwerfwindel umfasst zum Schließen des Hüftgürtels an wenigstens einem freien Ende des Hüftgürtels ein Verschlussmittel, welches ein wie vorausgehend beschrieben ausgebildetes Flachmaterial umfasst. Dieses Flachmaterial ist dann mit seinem ersten Bereich auf eine erste Oberfläche der Wegwerfwindel, insbesondere auf eine erste Oberfläche des Hüftgürtels aufgebracht. Dies kann durch an sich beliebige Fügeverfahren, insbesondere durch Ultraschallschweißverfahren, Thermobonding oder durch eine Kleberbeschichtung erfolgen.

Bei der Aufbringung auf einen Hüftgürtel erweist es sich als vorteilhaft, dass das Flachmaterial mit seinem ersten Bereich auf eine beim bestimmungsgemäßen Gebrauch körperabgewandt orientierte erste Oberfläche des Hüftgürtels aufgebracht ist.

Außerdem ist das Flachmaterial vermittels der ersten Kleberbeschichtung auf eine zweite Oberfläche des Hüftgürtels, und zwar für den bestimmungsgemäßen Gebrauch vorzugsweise unlösbar aufgebracht.

In Weiterbildung der Erfindung ist die zweite Oberfläche des Hüftgürtels der ersten Oberfläche gegenüberliegend angeordnet, so dass das Flachmaterial mit seinem ersten Bereich beispielsweise außen und mit dem Flachmaterialabschnitt dann innen am Hüftgürtel angeordnet ist. Dabei ist die Trägerschicht dann um Längsränder des Hüftgürtels herum um 180° gefaltet.

Nach einem weiteren Erfindungsgedanken ist dann die Trägerschicht von dem auf die zweite Oberfläche aufgebrachten Flachmaterialabschnitt ablösbar und vermittels einer Kleberbeschichtung, die auf dem zweiten Bereich der Trägerschicht verbleibt, als weiteres Verschlussmittel verwendbar. Durch das Ablösen der Trägerschicht von dem vorzugsweise unlösbar an die zweite Oberfläche des Hüftgürtels angefügten Flachmaterialabschnitt werden die mechanisch wirkenden Verschlusselemente freigegeben und können zur Schließung des Hüftgürtels eine lösbare Schließverbindung mit einem geeigneten Auftreffbereich, insbesondere einer textilen Oberfläche oder dort entsprechend vorgesehenen mechanisch wirkenden Gegenverschlusselementen am Hüftgürtel eingehen. Die zuvor von dem Flachmaterialabschnitt abgelöste Trägerschicht kann dann vermittels der Kleberbeschichtung als sekundäres Verschlussmittel verwendet werden, indem es beispielsweise einen Überlappungsbereich der freien Gürtelenden überfängt.

Es wäre aber auch denkbar, dass die Trägerschicht sowohl von dem Flachmaterialabschnitt als auch von der ersten Oberfläche ablösbar ist, so dass sie verworfen werden kann.

Es versteht sich, dass auch die zweiten Verschlussmittel am Windelhauptteil ein Flachmaterial der erfindungsgemäßen Art in vorteilhafter Weise umfassen können.

Nach einem bevorzugten Ausführungsbeispiel der erfindungsgemäßen Wegwerfwindel ist der Hüftgürtel von einem einstückigen Materialabschnitt gebildet, der an den Windelhauptteil angefügt ist und beidseits einer Längsmittelachse um in einer Längsrichtung der Windel erstreckte Falzlinien auf sich selbst gefaltet ist.

Es wird also erfindungsgemäß ein in Querrichtung oder Hüftumfangsrichtung einstückig durchgehender Materialabschnitt an den Windelhauptteil zur Bildung des Hüftgürtels angefügt. Aufgrund der flächenhaften Anfügung werden die Zugkräfte nicht in den Windelhauptteil eingeleitet sondern können ganz von dem reißfesten Gürtelmaterial aufgenommen werden. Dies eröffnet bei der Auswahl der den Hauptteil bildenden Chassismaterialien eine größere Flexibilität. Außerdem sind die Anforderungen an die Stabilität der Anfügung des Gürtels an den Hauptteil geringer. Der den Gürtel bildende Materialabschnitt kann, wie später anhand des erfindungsgemäßen Herstellungsverfahrens erörtert werden wird, als ein einziger Längsabschnitt einer in der Maschinenrichtung zugeführten Flachmaterialbahn erhalten werden.

In weiterer Ausbildung des Erfindungsgedankens erweist es sich als vorteilhaft, wenn der Hüftgürtel in der beidseits gefalteten Konfiguration lösbar fixiert ist, so dass er sich innerhalb der schnelllaufenden Herstellungsmaschine nicht unbeabsichtigt entfaltet oder aufflattert. Die Faltung und lösbare Fixierung des Hüftgürtels in der gefalteten Konfiguration ermöglicht in vorteilhafte Weise die Anfügung des sehr langen Hüftgürtels an den Hauptteil in der Herstellungsmaschine.

In Weiterbildung dieses Erfindungsgedankens erweist es sich als vorteilhaft, wenn die lösbare Fixierung durch Fügestellen oder Fügebereiche zwischen den aufeinandergefalteten Teilabschnitten des den Hüftgürtel bildenden Materialabschnitts gebildet ist. Es wäre aber auch möglich, das die gefaltete Konfiguration durch sonstige Haltemittel, etwa einen lösbaren Tapeabschnitt, lösbar fixiert ist.

Die vorerwähnte und vorzugsweise in einem Zug zu lösende Fixierung der aufeinander gefalteten Teilabschnitte des an den Hauptteil angefügten den Hüftgürtel bildenden Materialabschnitts kann beispielsweise durch kalte Verprägung, oder durch Verprägung unter Anwendung von Temperatur (Thermoverschweißung), durch Vernadelung, insbesondere Heißvernadelung, oder durch Ultraschallverschweißung oder Laserverschweißung oder ähnliche gleichwirkende Fügemethoden erreicht werden.

Im einfachsten Fall ist der Materialabschnitt beidseits der Längsmittelachse um eine Falzlinie auf sich selbst gefaltet, so dass zwei Teilabschnitte aufeinander liegen bzw. gegeneinander anliegen. Vorzugsweise ist der Materialabschnitt aber um wenigstens zwei Falzlinien auf sich selbst gefaltet, so dass eine im Schnitt Z-förmige Konfiguration entsteht. Nach einer weiteren bevorzugten Ausführungsform sind die Materialabschnitte um drei Falzlinien auf sich selbst gefaltet. Nach einer weiteren bevorzugten Ausführungsform sind die Materialabschnitte um vier Falzlinien auf sich selbst gefaltet.

Die Gürtelwindel erweist sich insbesondere bei der Anwendung bei stark pflegebedürftigen Personen als besonders vorteilhaft. So wird die Gürtelwindel beispielsweise häufig an pflegebedürftige Patienten angelegt, während diese sich in Seitenlage befinden. Hierbei muss der über den Hauptteil seitlich überstehende Materialabschnitt unter dem Patienten hindurch geführt werden. Dieser Vorgang des unter dem Patienten Hindurchführens ist mit dem lösbar fixierten gefalteten Materialabschnitt, welcher den Gürtel bildet, nun wesentlich vereinfacht.
Die lösbare Fixierung der aufeinander gefalteten Teilabschnitte des Materialabschnitts aneinander und möglicherweise auch an dem Hauptteil ist vorzugsweise durch mehrere im Wesentlichen punktförmige Fügestellen ausgebildet. Eine punktförmige Fügestelle der vorstehend erwähnten Art bedeutet, dass die Fügestelle eine Fläche (in Projektion auf die X-Y-Ebene des Hauptteils) von weniger als 5 mm², insbesondere von weniger als 2 mm² und weiter insbesondere von weniger als 1 mm² aufweist. Die Fügestellen müssen nicht streng punkt- oder kreisförmig sein. Denkbar und vorteilhaft sind auch von der Punkt- oder Kreisform abweichende Formen wie Dreieck-, Viereck-, Vieleck-, oder Ovalformen. Vorzugsweise ist die lösbare Fixierung der aufeinander gefalteten Teilabschnitte der Materialabschnitte aneinander durch thermisch oder durch Ultraschall erzeugte, vorzugsweise punktförmige Fügestellen ausgebildet.

Weitere Merkmale, Einzelheiten und Vorteile der Erfindung ergeben sich aus den beigefügten Patentansprüchen und der zeichnerischen Darstellung und nachfolgenden Beschreibung einer bevorzugten Ausführungsform des erfindungsgemäßen Flachmaterials und einer erfindungsgemäßen Wegwerfwindel.

In der Zeichnung zeigt:
- Figur 1: eine schematische Schnittansicht (nicht maßstabsgetreu) durch ein erfindungsgemäßes Flachmaterial;
- Figur 2: eine Draufsicht auf das Flachmaterial nach Figur 1;
- Figur 3: eine Schnittansicht des Flachmaterials nach Figur 1 in einem applizierten Zustand und
- Figur 4: eine Schnittansicht des Flachmaterials nach Figur 1 in einem applizierten Zustand nach dem Lösen der Trägerschicht von dem Flachmaterialabschnitt
- Figur 5: eine perspektivische Ansicht des Flachmaterials in endloser Rollenform;
- Figur 6: eine schematische Ansicht einer Wegwerfwindel mit einem Hüftgürtel;
- Figur 7: eine Schnittansicht mit Schnittebene A-A aus Figur 6;
- Figur 8: eine Figur 7 entsprechende Schnittansicht mit einer anderen Gürtellänge.

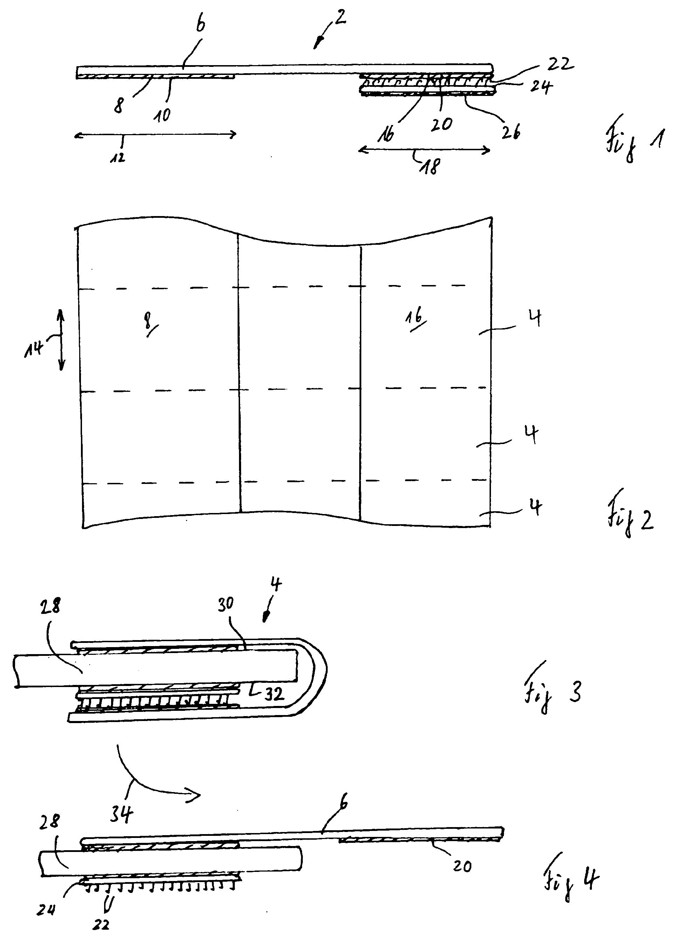

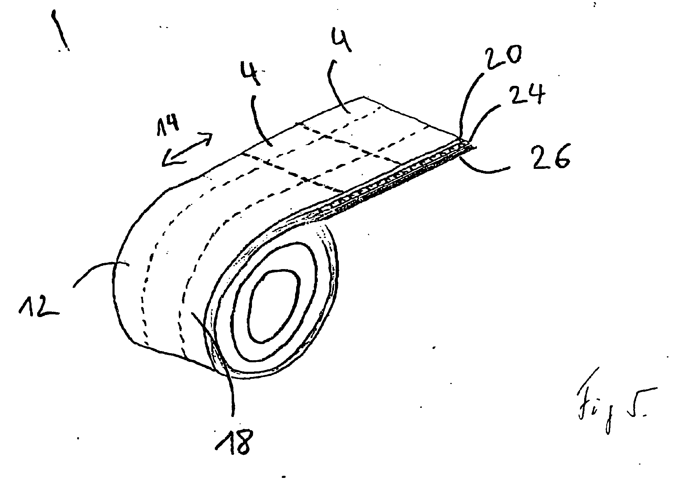

Figur 1 zeigt eine Schnittansicht durch ein insgesamt mit dem Bezugszeichen 2 bezeichnetes Flachmaterial. Längsabschnitte 4 dieses Flachmaterials, wie sie in Figur 2 angedeutet sind, lassen sich zur Bildung von Verschlussmitteln bei wegwerfbaren Hygieneartikeln verwenden. Das Flachmaterial umfasst eine erste Trägerschicht 6 mit einem ersten Bereich 8, auf den eine Kleberbeschichtung 10 aufgebracht ist. Die Erstreckung des ersten Bereichs 8 in der dargestellten Schnittebene ist mit dem Bezugszeichen 12 bezeichnet. Die Erstreckung in der aus Figur 2 ersichtlichen Längsrichtung 14 ist endlos. Es wäre aber auch denkbar, dass das Flachmaterial eine Vielzahl diskreter erster Bereiche mit diskretem Kleberauftrag aufweist, wobei der aus Figur 2 angedeutete Schnitt insbesondere durch kleberfreie Regionen geführt werden kann.

Die Trägerschicht 6 kann von einer Folie, einem Vlies oder einem Vlies/Folienlaminat gebildet sein.

Des Weiteren umfasst das Flachmaterial 2 einen zweiten Bereich 16, der im dargestellten Fall von dem ersten Bereich 8 beabstandet ist, aber auch im Wesentlichen unmittelbar hieran angrenzen könnte. Die Quererstreckung des zweiten Bereichs 16 ist mit dem Bezugszeichen 18 bezeichnet. Seine Erstreckung in Längsrichtung 14 ist wiederum endlos. Auch der zweite Bereich trägt eine Kleberbeschichtung 20. Mittels dieser Kleberbeschichtung 20 haftet ein mechanische Verschlusselemente 22 aufweisender Flachmaterialabschnitt 24 an der Trägerschicht 6. Der Flachmaterialabschnitt 24 ist dabei so orientiert, dass er mit seinen mechanisch wirkenden Verschlusselementen 22 zur Kleberbeschichtung 20 und damit zur Trägerschicht 6 hin orientiert ist. Er haftet also mit der Seite der mechanischen Verschlusselemente 22 mittels der Kleberbeschichtung 20 an der Trägerschicht 6. Der Flachmaterialabschnitt 24, der im dargestellten Fall auch endlos auf den zweiten Bereich 16 der Trägerschicht 6 aufgebracht ist, weist auf seiner von der Trägerschicht 6 abgewandten Seite eine Kleberbeschichtung 26 auf.

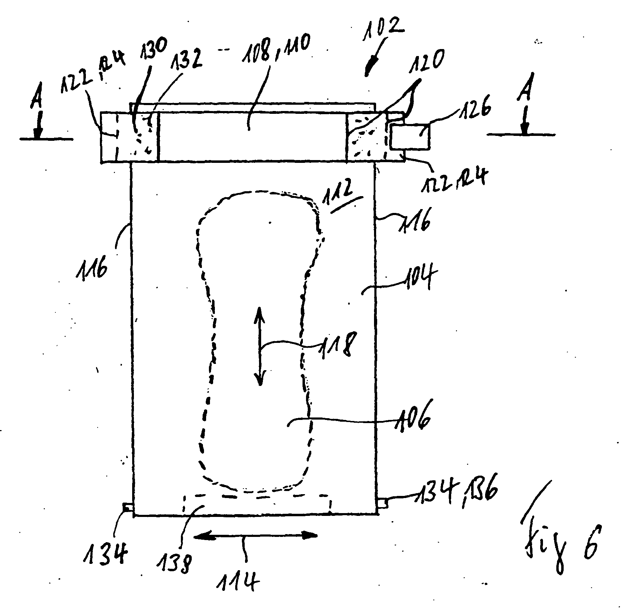

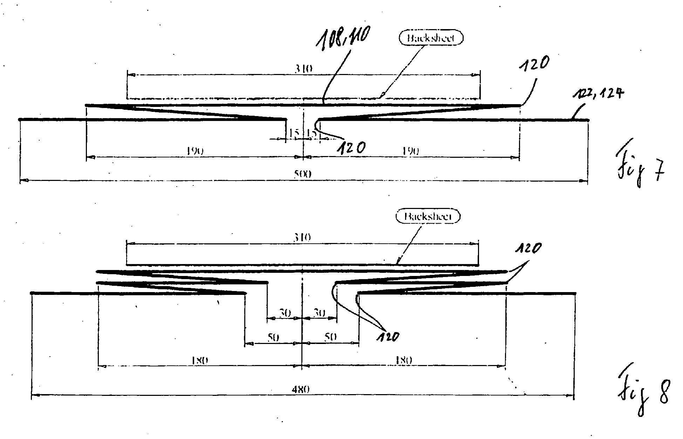

Figur 3 zeigt eine mögliche Applikation eines Längsabschnitts 4 des Flachmaterials 2 an einem angedeuteten Seitenrandbereich 28 eines Hygieneartikels, insbesondere und vorzugsweise an einem Hüftgürtel einer Wegwerfwindel. Im dargestellten Fall ist der Längsabschnitt 4 mit der Kleberbeschichtung 10 des ersten Bereichs 8 auf eine erste Oberfläche 30 des Längsrandbereichs 28 aufgebracht und mit der Kleberbeschichtung 26 des Flachmaterialabschnitts 24 auf eine zweite Oberfläche 32 des Längsrandbereichs 28. Die Haftverbindung gegen die zweite Oberfläche 32 ist dabei stärker ausgebildet als die Haftverbindung zwischen dem Flachmaterialabschnitt 24 und dem zweiten Bereich 16 unter Vermittlung der Kleberbeschichtung 20. Wird ausgehend von der in Figur 3 dargestellten applizierten Situation die Trägerschicht in Richtung des Pfeils 34 abgelöst, so löst sich die Trägerschicht 6 mit ihrem zweiten Bereich 16 von dem Flachmaterialabschnitt 24, der aufgrund der größeren Haftkraft zur zweiten Oberfläche 32 daran verbleibt. Somit werden die mechanisch wirkenden Verschlusselemente 22 des Flachmaterialabschnitts 24 exponiert, so wie dies in Figur 4 dargestellt ist. Der Längsrandbereich 28, beispielsweise eines Hüftgürtels, könnte nun unter Verwendung der Verschlusselemente 22 des Flachmaterialabschnitts 24 auf einen entsprechend ausgebildeten Auftreffbereich, insbesondere das andere Gürtelende aufgebracht werden, um dort eine mechanisch wirkende Schließfunktion auszuüben. Dabei könnte die Trägerschicht 6 als weiteres Sekundärverschlusselement dienen, indem es mit seiner Kleberbeschichtung 20 ebenfalls auf einen Landebereich lösbar klebend haftend aufgebracht wird.

Figur 6 zeigt schematisch eine wegwerfbare Gürtelwindel 102 mit einem Hauptteil 104 und einem angedeuteten Absorptionskörper 106. An den Hauptteil 104 angefügt ist ein einstückiger Materialabschnitt 108, welcher einen Hüftgürtel 110 der Gürtelwindel bildet. Der einstückige Materialabschnitt 108 ist an eine Außenseite 112 des Hauptteils 104 auf noch näher zu beschreibende Weise unlösbar angefügt. Er erstreckt sich in Querrichtung 114 der Gürtelwindel 102 über seitliche Längsränder 116 im entfalteten Zustand über wenigstens 300 mm, insbesondere über wenigstens 400 mm, insbesondere über wenigstens 500 mm, insbesondere über wenigstens 600 mm hinaus. Im dargestellten Fall der Figur 6 ist der einstückige Materialabschnitt 108 beidseits einer Windellängsrichtung 118 um Falzlinien 120 mehrfach auf sich selbst gefaltet. Das jeweils freie Ende 122 des Materialabschnitts 108 bildet einen über die Längsseitenränder 116 um wenigstens 10 mm vorstehenden Anfassbereich 124 zum Ergreifen mit den Fingern eines Benutzers. Dort ist auch ein Verschlussmittel 126, das als Längsabschnitt 4 des vorstehend beschriebenen Flachmaterials 2 gebildet ist, an einem freien Ende 122 vorgesehen, welches mit einem Auftreffbereich oder Gegenverschlusselementen am anderen Ende 122 des Hüftgürtels 110 lösbar haftend zusammenwirken kann, wenn der Hüftgürtel 110 zur Bildung einer in Hüftumfangsrichtung geschlossenen Hüftöffnung geschlossen wird.

Die aufeinander gefalteten Teilabschnitte 130 des einstückigen Materialabschnitts 108 sind lösbar aufeinander fixiert, und zwar durch eine Anzahl von im Wesentlichen punktförmigen Fügestellen 132 in Form von vorzugsweise Ultraschallschweißpunkten.

Zum Anlegen der Gürtelwindel 102 wird der Hüftgürtel 110 entfaltet und über das Verschlussmittel 126 und den entsprechend ausgebildeten Auftreffbereich am anderen freien Ende des Hüftgürtels 10 auf sich selbst geschlossen. Der Benutzer oder eine Pflegeperson holt nun das im allgemeinen frei nach unten hängende Ende des Hauptteils zwischen den Beinen des Benutzers hervor und befestigt es lösbar an dem ringförmig geschlossenen Hüftgürtel 110, und zwar vorzugsweise auf der vom Benutzer abgewandten Außenseite des Hüftgürtels 110. Der Hauptteil 104 kann wiederum über an sich beliebige klebend oder mechanisch wirkende Verschlussmittel 134, im dargestellten Fall über seitlich vom Hauptteil vorstehende Verschlusslaschen 136 lösbar festgelegt werden. Zusätzlich können in einem entsprechenden Überdeckungsbereich 138, vorzugsweise an der körperzugewandten Innenseite des Hauptteils 104, Verschlussmittel im weitesten Sinn und in an sich beliebiger Ausführung vorgesehen sein.

Die Figuren 7 und 8 zeigen verschiedene Faltungen des einstückigen Materialabschnitts 108 (ohne Darstellung des Verschlussmittels 126). Die Faltung nach Figur 7 umfasst zwei Falzlinien 120 auf jeder Seite; eine jeweilige Faltung ist also Z-förmig. Die Gesamtlänge des Hüftgürtels 110 (einschließlich der Quererstreckung über den Hauptteil, also die Umfangslänge der gesamten Hüftöffnung) beträgt ca. 1200 mm (ein etwaiger Überlappungsbereich beim Schließen des Hüftgürtels 110 ist dabei nicht berücksichtigt). Die Gürtelbreite, also deren Erstreckung in Längsrichtung 118, beträgt zwischen 50 und 100 mm.

Die in Figur 8 dargestellte Faltung umfasst auf jeder Seite vier Falzlinien 20. Die gesamte Gürtellänge beträgt ca. 1600 mm.

## Patentansprüche

1. Flachmaterial (2), insbesondere zur Bildung von Verschlussmitteln bei einem wegwerfbaren Hygieneartikel, mit einer ersten Trägerschicht (6) mit einem ersten Bereich (8) zur Anbringung des Flachmaterials auf einer ersten Oberfläche (30) und mit einem zweiten Bereich (16), und mit einem flächenhaft erstreckten, mechanische Verschlusselemente (22), die von einer hakenbildenden Komponente eines Haken/Schlaufenmaterials gebildet sind, aufweisenden Flachmaterialabschnitt (24), der mit diesen Verschlusselementen (22) dem zweiten Bereich (16) zugewandt orientiert ist und lösbar an den zweiten Bereich angefügt ist, wobei der Flachmaterialabschnitt (24) auf seiner von dem zweiten Bereich (16) abgewandten Oberfläche eine erste Kleberbeschichtung (26) aufweist, mit der er auf eine zweite der ersten Oberfläche (30) gegenüberliegende Oberfläche (32) appliziert werden kann, **dadurch gekennzeichnet, dass** der flächenhaft erstreckte, Flachmaterialabschnitt (24) mit der Seite der mechanischen Verschlusselemente (22) mittels einer Kleberbeschichtung (20) lösbar an dem zweiten Bereich (16) haftet, dass die erste Kleberbeschichtung (26) eine größere Haftkraft zwischen dem Flachmaterialabschnitt (24) und der zweiten der ersten Oberfläche (30) gegenüberliegenden Oberfläche (32) ausübt als die Haftverbindung zwischen dem Flachmaterialabschnitt (24) und dem zweiten Bereich (16) unter Vermittlung der Kleberbeschichtung (20), so dass die hakenbildende Komponente auf dieser zweiten Oberfläche (32) verbleibt und eine lösbare Schließverbindunq mit einem geeigneten Auftreffbereich eingehen kann und die Trägerschicht (6) mit der Kleberbeschichtung (20) ein weiteres Sekundärverschlusselement bildet.

2. Flachmaterial nach Anspruch 1, **dadurch gekennzeichnet, dass** der erste Bereich (8) eine Kleberbeschichtung (10) aufweist.

3. Flachmaterial nach Anspruch 1 oder 2, **dadurch gekennzeichnet, dass** es biegsam und flexibel beschaffen ist.

4. Flachmaterial nach einem oder mehreren der vorstehenden Ansprüche, **dadurch gekennzeichnet, dass** die erste Trägerschicht (6) von einem Vliesstoff, einer Folie oder einem Vlies/Folien-Verbundwerkstoff gebildet ist.

5. Flachmaterial nach einem oder mehreren der vorstehenden Ansprüche in einer übereinander angeordneten Konfiguration, wobei die erste Kleberbeschichtung (26) des Flachmaterialabschnitts (24) einer Bahn auf einer von dem Flachmaterialabschnitt (24) abgewandten Seite der Trägerschicht (6) einer angrenzenden Bahn zu liegen kommt.

6. Flachmaterial nach Anspruch 5, in Endlosform und auf eine Rolle aufgewickelt.

7. Flachmaterial nach einem oder mehreren der vorstehenden Ansprüche, **dadurch gekennzeichnet, dass** die Trägerschicht (6) auf ihrer dem Flachmaterialabschnitt (24) abgewandten Seite eine Release-Beschichtung aufweist.

8. Wegwerfwindel (102), insbesondere zur Inkontinentenversorgung, mit einem eine in Umfangsrichtung geschlossene Hüftöffnung der Windel bildenden Hüftgürtel (110), der an wenigstens einer Stelle öffenbar und auf sich selbst schließbar ist, wobei an einem in Querrichtung (114) freien Ende des Hüftgürtels (110) ein erstes Verschlussmittel (126) vorgesehen ist, und mit einem einen Vorderbereich, einen Rückenbereich und einen dazwischenliegenden Schrittbereich aufweisenden Windelhauptteil (104), der einen Absorptionskörper (106) für Flüssigkeiten aufweist, wobei der Windelhauptteil (104) mit dem Längsende seines Vorderbereichs oder seines Rückenbereichs über zweite Verschlussmittel (134, 136) lösbar am Hüftgürtel (110) festlegbar ist, derart, dass ein Benutzer bei angelegtem Hüftgürtel den Windelhauptteil (104) zwischen den Beinen hervorholen und das noch freie Längsende des Windelhauptteils (104) am Hüftgürtel (110) lösbar festlegen kann, **dadurch gekennzeichnet, dass** das erste Verschlussmittel (126) ein Flachmaterial (2) nach einem oder mehreren der vorstehenden Ansprüche 1 - 7 umfasst.

9. Wegwerfwindel nach Anspruch 8, **dadurch gekennzeichnet, dass** das Flachmaterial (2) mit seinem ersten Bereich (8) auf eine erste Oberfläche (30) des Hüftgürtels (110) aufgebracht ist.

10. Wegwerfwindel nach Anspruch 8 oder 9, **dadurch gekennzeichnet, dass** das Flachmaterial (2) mit seinem ersten Bereich (8) auf eine beim bestimmungsgemäßen Gebrauch körperabgewandt orientierte erste Oberfläche (30) des Hüftgürtels (110) aufgebracht ist.

11. Wegwerfwindel nach Anspruch 10, **dadurch gekennzeichnet, dass** das Flachmaterial (2) mit der ersten Kleberbeschichtung (26) auf eine zweite Oberfläche (32) des Hüftgürtels (110) unlösbar aufgebracht ist.

12. Wegwerfwindel nach Anspruch 11, **dadurch gekennzeichnet, dass** die zweite Oberfläche (32) des Hüftgürtels (110) der ersten Oberfläche (30) gegenüberliegend angeordnet ist.

13. Wegwerfwindel nach einem der Ansprüche 8-12, **dadurch gekennzeichnet, dass** die Trägerschicht (6) von dem auf die zweite Oberfläche (32) aufgebrachten Flachmaterialabschnitt (24) ablösbar ist und vermittels einer Kleberbeschichtung (20) auf dem zweiten Bereich (16) als weiteres Verschlussmittel verwendbar ist.

14. Wegwerfwindel nach einem der Ansprüche 8-13, **dadurch gekennzeichnet, dass** die Trägerschicht (6) von dem auf die zweite Oberfläche (32) aufgebrachten Flachmaterialabschnitt (24) und auch von der ersten Oberfläche (30) ablösbar ist, so dass sie verworfen werden kann.

15. Wegwerfwindel nach einem der Ansprüche 8-14, **dadurch gekennzeichnet, dass** auch die zweiten Verschlussmittel (134, 136) am Windelhauptteil (104) mit einem Flachmaterial (2) nach einem oder mehreren der Ansprüche 1 - 7 vorgesehen sind.

16. Wegwerfwindel nach einem der Ansprüche 8-15, **dadurch gekennzeichnet, dass** der Hüftgürtel (110) von einem einstückigen Materialabschnitt (108) gebildet ist, der an den Windelhauptteil (104) angefügt ist und beidseits einer Längsmittelachse (118) um in einer Längsrichtung der Windel erstreckte Falzlinien (120) auf sich selbst gefaltet ist.

17. Wegwerfwindel nach einem der Ansprüche 8-16, **dadurch gekennzeichnet, dass** der Hüftgürtel (110) in der gefalteten Konfiguration lösbar fixiert ist.

18. Wegwerfwindel nach Anspruch 17, **dadurch gekennzeichnet, dass** die lösbare Fixierung durch Fügestellen (132) oder Fügebereiche zwischen den aufeinander gefalteten Teilabschnitten (130) des den Hüftgürtel bildenden Materialabschnitts (108) gebildet ist.

19. Wegwerfwindel nach Anspruch 18, **dadurch gekennzeichnet, dass** die Fügestellen (132) oder Fügebereich im wesentlichen punktförmig sind.

## Claims

1. Planar material (2), in particular for forming closure means for a disposable sanitary item, having a first carrier layer (6) with a first region (8) for applying the planar material to a first surface (30) and with a second region (16), and having a planar material portion (24) extending two-dimensionally, and comprising mechanical closure elements (22) which are formed from a hook-forming component of a hook/loop material, which planar material portion with these closure elements (22) faces the second region (16) and is detachably joined to the second region (16), the planar material portion (24) having a first adhesive coating (26) on its surface remote from the second region (16), with which coating it can be attached to a second surface (32) opposing the first surface (30), **characterised in that** the planar material portion (24) extending two-dimensionally detachably adheres with the side of the mechanical closure elements (22) to the second region (16) by means of an adhesive coating (20), **in that** the first adhesive coating (26) exerts a stronger adhesive force between the planar material portion (24) and the second surface (32) opposing the first surface (30) than the adhesion by means of the adhesive coating (20) between the planar material portion (24) and the second region (16) so that the hook-forming component remains on this second surface (32) and can enter into a detachable closing fit with an appropriate striking region and the carrier layer (6) with the adhesive coating (20) forms an additional secondary closure element.

2. Planar material according to claim 1, **characterised in that** the first region (8) has an adhesive coating (10).

3. Planar material according to either claim 1 or claim 2, **characterised in that** the material is pliable and flexible.

4. Planar material according to one or more of the preceding claims, **characterised in that** the first carrier layer (6) is formed from a non-woven material, a film or a non-woven/film compound material.

5. Planar material according to one or more of the preceding claims in a superimposed configuration, wherein the first adhesive coating (26) of the planar material portion (24) of a strip rests on a side of the carrier layer (6), remote from the planar material portion (24), of an adjacent strip.

6. Planar material according to claim 5, in continuous form and wound around a roller.

7. Planar material according to one or more of the preceding claims, **characterised in that** the carrier layer (6) has a release coating on its side remote from the planar material portion (24).

8. Disposable pad (102), in particular for incontinence, comprising a belt (110) forming an opening in the pad closed in a circumferential direction, which can opened in at least one position and can be closed on itself, wherein at a free end of the belt (110) in a transverse direction (114) a first closure means (126) is provided, and comprising a pad body (104) having a leading region, a trailing region and an intermediate crotch region disposed therebetween, which pad body has an absorption core (106) for liquids, wherein the pad body (104) can be fixed detachably to the belt (110) with the longitudinal end of its leading region or its trailing region via two closure means (134, 136) in such a way that a user can place the pad body (104) between his legs when wearing the belt and the longitudinal end of the pad body (104) that is still free can be detachably fixed to the belt (110), **characterised in that** the first closure means (126) comprises a planar material (2) according to one or more of preceding claims 1 to 7.

9. Disposable pad according to claim 8, **characterised in that** the planar material (2) with its first region (8) is attached to a first surface (30) of the belt (110).

10. Disposable pad according to either claim 8 or claim 9, **characterised in that** the planar material (2) with its first region (8) is attached to a first surface (30) of the belt (110) which is remote from the body when used as intended.

11. Disposable pad according to claim 10, **characterised in that** the planar material (2) with the first adhesive coating (26) is attached to a second surface (32) of the belt (110) such that it cannot be detached.

12. Disposable pad according to claim 11, **characterised in that** the second surface (32) of the belt (110) opposes the first surface (30).

13. Disposable pad according to any one of claims 8 to 12, **characterised in that** the carrier layer (6) can be detached from the planar material portion (24) attached to the second surface (32) and can be used as a further closure means by way of a coating (20) for adhering to the second region (16).

14. Disposable pad according to any one of claims 8 to 13, **characterised in that** the carrier layer (6) can be detached from the planar material portion (24) attached to the second surface (32) and also from the first surface (30) so that said carrier layer can be disposed of

15. Disposable pad according to any one of claims 8 to 14, **characterised in that** the two closure means (134, 136) on the pad body (104) are also provided with a planar material (2) according to one or more of claims 1 to 7.

16. Disposable pad according to any one of claims 8 to 15, **characterised in that** the belt (110) is formed from a portion of material (108) in one piece, which is joined to the pad body (104) and is folded in on itself about fold lines (120) extending in a longitudinal direction of the pad on both sides of a longitudinal centre axis (118).

17. Disposable pad according to any one of claims 8 to 16, **characterised in that** the belt (110) is detachably fixed in the folded configuration.

18. Disposable pad according to claim 17, **characterised in that** the detachable fixing is formed by joining points (132) or joining regions between the partial portions (130) folded in on one another of the portion (108) of material forming the belt (110).

19. Disposable pad according to claim 18, **characterised in that** the joining points (132) or joining region are substantially punctiform.

## Revendications

1. Matière plate (2), en particulier destinée à la formation de moyens de fermeture pour un article d'hygiène jetable, comportant une première couche de support (6) ayant une première zone (8) pour l'application de la matière plate sur une première surface (30) et une seconde zone (16), et comportant un segment de matière plate (24) s'étendant à plat et présentant des éléments de fermeture mécaniques (22) qui sont formés par une composante formant des crochets d'une matière bouclée ou à crochets, lequel segment de matière plate est orienté avec ces éléments de fermeture (22) tourné vers la seconde zone (16) et est assemblé de manière amovible à la seconde zone, le segment de matière plate (24) présentant sur sa surface orientée à l'opposé de la seconde zone (16) un premier revêtement adhésif (26) avec lequel il peut être appliqué sur une seconde surface (32) opposée à la première surface (30), **caractérisée en ce que** le segment de matière plate (24) s'étendant à plat adhère de manière amovible avec le côté des éléments de fermeture mécaniques (22) au moyen d'un revêtement adhésif (20) sur la seconde zone (16), **en ce que** le premier revêtement adhésif (26) exerce, entre le segment de matière plate (24) et la seconde surface (32) opposée à la première surface (30), une adhérence supérieure à la liaison adhésive entre le segment de matière plate (24) et la seconde zone (16) obtenue par le revêtement adhésif (20), de sorte que la composante formant des crochets reste sur cette seconde surface (32) et peut former une liaison de fermeture amovible avec une zone d'incidence appropriée et la couche de support (6) forme un autre élément de fermeture secondaire avec le revêtement adhésif (20).

2. Matière plate selon la revendication 1, **caractérisée en ce que** la première zone (8) présente un revêtement adhésif (10).

3. Matière plate selon la revendication 1 ou 2, **caractérisée en ce que** sa constitution est souple et flexible.

4. Matière plate selon l'une ou plusieurs des revendications précédentes, **caractérisée en ce que** la première couche de support (6) est formée par un non-tissé, un film ou une matière composite non-tissé/film.

5. Matière plate selon l'une ou plusieurs des revendications précédentes dans une configuration d'agencement superposé, le premier revêtement adhésif (26) du segment de matière plate (24) d'une bande venant reposer sur une face, opposée au segment de matière plate (24), de la couche de support (6) d'une bande contiguë.

6. Matière plate selon la revendication 5, sous une forme sans fin et enroulée sur un rouleau.

7. Matière plate selon l'une ou plusieurs des revendications précédentes, **caractérisée en ce que** la couche de support (6) présente un revêtement antiadhésif sur sa face orientée à l'opposé du segment de matière plate (24).

8. Couche jetable (102), en particulier pour les soins aux incontinents, comportant une ceinture de taille (110) formant une ouverture de taille de la couche, fermée dans le sens périphérique, laquelle ceinture de taille peut être ouverte à au moins un endroit et fermée sur elle-même, un premier moyen de fermeture (126) étant prévu à une extrémité libre de la ceinture de taille (110) dans le sens transversal (114), et comportant une partie principale de couche (104) présentant une zone avant, une zone arrière et une zone d'entre-jambes située entre celles-ci, laquelle partie principale de couche présente un corps d'absorption (106) pour les liquides, la partie principale de couche (104) pouvant être fixée de manière amovible à la ceinture de taille (110) avec l'extrémité longitudinale de sa zone avant ou de sa zone arrière par le biais de seconds moyens de fermeture (134, 136), de telle manière qu'un utilisateur puisse ramener entre les jambes la partie principale de couche (104) lorsque la ceinture de taille est mise et fixer de manière amovible l'extrémité longitudinale encore libre de la partie principale de couche (104) sur la ceinture de taille (110), **caractérisée en ce que** le premier moyen de fermeture (126) comprend une matière plate (2) selon l'une ou plusieurs des revendications précédentes 1 à 7.

9. Couche jetable selon la revendication 8, **caractérisée en ce que** la matière plate (2) est placée avec sa première zone (8) sur une première surface (30) de la ceinture de taille (110).

10. Couche jetable selon la revendication 8 ou 9, **caractérisée en ce que** la matière plate (2) est placée avec sa première zone (8) sur une première surface (30) de la ceinture de taille (110), orientée à l'opposé du corps lors de l'utilisation conforme à la définition.

11. Couche jetable selon la revendication 10, **caractérisée en ce que** la matière plate (2) est placée de manière non détachable avec le premier revêtement adhésif (26) sur une seconde surface (32) de la ceinture de taille (110).

12. Couche jetable selon la revendication 11, **caractérisée en ce que** la seconde surface (32) de la ceinture de taille (110) est agencée à l'opposé de la première surface (30).

13. Couche jetable selon l'une des revendications 8 à 12, **caractérisée en ce que** la couche de support (6) est détachable du segment de matière plate (24) appliqué sur la seconde surface (32) et peut être utilisée comme autre moyen de fermeture par le biais d'un revêtement adhésif (20) sur la seconde zone (16).

14. Couche jetable selon l'une des revendications 8 à 13, **caractérisée en ce que** la couche de support (6) est détachable du segment de matière plate (24) appliqué sur la seconde surface (32) et également de la première surface (30), de sorte qu'elle peut être jetée.

15. Couche jetable selon l'une des revendications 8 à 14, **caractérisée en ce que** les seconds moyens de fermeture (134, 136) sur la partie principale de couche (104) sont également prévus avec une matière plate (2) selon l'une ou plusieurs des revendications 1 à 7.

16. Couche jetable selon l'une des revendications 8 à 15, **caractérisée en ce que** la ceinture de taille (110) est formée par un segment de matière (108) d'une seule pièce, lequel est assemblé à la partie principale de couche (104) et plié sur lui-même des deux côtés d'un axe médian longitudinal (118) autour de lignes de pliage (120) s'étendant dans un sens longitudinal de la couche.

17. Couche jetable selon l'une des revendications 8 à 16, **caractérisée en ce que** la ceinture de taille (110) est fixée de manière amovible dans la configuration pliée.

18. Couche jetable selon la revendication 17, **caractérisée en ce que** la fixation amovible est formée par des points d'assemblage (132) ou zones d'assemblage entre les segments partiels (130) pliés l'un sur l'autre du segment de matière (108) formant la ceinture de taille.

19. Couche jetable selon la revendication 18, **caractérisée en ce que** les points d'assemblage (132) ou zones d'assemblage sont essentiellement sous forme de points.
